(19) Europäisches Patentamt
European Patent Office
Office européen des brevets

(11) **EP 4 663 658 A1**

(12) **EUROPEAN PATENT APPLICATION**
published in accordance with Art. 153(4) EPC

(43) Date of publication:
**17.12.2025 Bulletin 2025/51**

(21) Application number: **23924738.0**

(22) Date of filing: **30.03.2023**

(51) International Patent Classification (IPC):
*C07K 16/28* (2006.01)      *C12N 15/13* (2006.01)
*C12N 15/85* (2006.01)      *C12N 5/10* (2006.01)
*A61K 47/68* (2017.01)      *A61K 49/00* (2006.01)
*A61K 51/10* (2006.01)      *G01N 33/574* (2006.01)

(86) International application number:
**PCT/CN2023/084931**

(87) International publication number:
**WO 2024/178772 (06.09.2024 Gazette 2024/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.02.2023   CN 202310173278**

(71) Applicant: **Carbiogene Therapeutics Co., Ltd.
Hangzhou, Zhejiang 310051 (CN)**

(72) Inventors:
• **YANG, Xin
Hangzhou, Zhejiang 310051 (CN)**
• **ZHU, Jiangao
Hangzhou, Zhejiang 310051 (CN)**
• **YANG, Wenjun
Hangzhou, Zhejiang 310051 (CN)**

(74) Representative: **Isarpatent
Patent- und Rechtsanwälte
Barth Hassa Peckmann & Partner mbB
Friedrichstraße 31
80801 München (DE)**

Remarks:
The complete document including Reference
Table(s) and the Sequence Listing(s) can be
downloaded from the EPO website

(54) **SINGLE-DOMAIN ANTIBODY SPECIFICALLY BINDING TO CLL1 PROTEIN, AND USE THEREOF**

(57)    The application discloses single-domain antibodies binding to the CLL1 protein and their uses. Specifically, it discloses a single-domain antibody with the amino acid sequence of SEQ ID No.1. This single-domain antibody exhibits high affinity and can effectively and specifically target CLL1-positive cells. It can efficiently promote the secretion of IFNγ by NK cells, activate the effector functions of NK cells, and significantly induce the cytotoxic effects of NK cells, thereby demonstrating a strong capacity for specifically killing CLL1-positive cells. The single-domain antibody of the present disclosure can be formulated into drugs for the clinical prevention or treatment of diseases related to the CLL1 target. It can also be used for the preparation of CAR cells targeting CLL1 or detection kits for the CLL1 protein. The single-domain antibody drug of the present disclosure features stable structure, small molecular size, ease of recombinant expression, and low production costs. It can be used alone or serve as a drug delivery system to carry relevant drugs, holding great promise and significant importance in fields such as pharmaceutical uses and clinical diagnosis.

EP 4 663 658 A1

## Description

### TECHNICAL FIELD

The present disclosure belongs to the field of biopharmaceuticals, specifically relating to a single-domain antibody that specifically bind to a CLL1 protein and use thereof.

### BACKGROUND

Acute Myeloid Leukemia (AML) is a clonal malignant proliferative disease of myeloid progenitor cells within the hematopoietic system, representing a highly heterogeneous group of diseases. It can arise from the malignant transformation of hematopoietic progenitor cells at various stages during normal myeloid cell differentiation and development. For a long time, anthracyclines combined with cytarabine chemotherapy have been employed to treat mild cases, while hematopoietic stem cell transplantation has been used for intermediate- and high-risk patients. The heterogeneity of AML poses a significant challenge to current treatment approaches for AML patients. In recent years, researchers have discovered that immune-targeted therapies targeting CLL1 have shown remarkable efficacy in treating AML. C-type lectin-like molecule 1 (CLL1), also known as C-type lectin domain family 12 member A (CLEC12A), is a type II transmembrane glycoprotein that plays a crucial role in immune regulation as an inhibitory receptor. CLL1 is present in myeloid cells in the peripheral blood and bone marrow, as well as in most AML cells. It is also expressed on the CD34+CD38- stem cells of most AML patients, whereas normal CD34$^+$CD38$^-$ stem cells do not express CLL1. Due to its unique expression pattern, CLL1 has emerged as a potential target for AML treatment and diagnosis. Additionally, CLL1 is expressed on cells of Myelodysplastic Syndromes (MDS) and Chronic Myeloid Leukemia (CML, also known as chronic granulocytic leukemia).

Antibodies are globulins synthesized and secreted by plasma cells differentiated and proliferated from B cells upon antigen stimulation. They specifically bind to antigens and possess various immune functions. The specific binding ability of antibodies to antigens renders them of great significance in disease diagnosis and immune prevention and treatment. Antibody drugs, composed of antibody substances (comprising full antibody molecules and antibody fragments with therapeutic functions), are an important means of targeted therapy and have become one of the most promising and valuable areas in the current biopharmaceutical industry. However, the large molecular size and strong antigenicity of antibody drugs hinder their widespread use in the pharmaceutical field.

Heavy chain antibodies (HcAbs) are a naturally occurring type of antibody found in camelids and cartilaginous fish. They uniquely lack light chains in their antibody domains and consist solely of two heavy chains. The antigen recognition function of HcAbs is primarily determined by the variable region of the heavy chain (VHH). The VHH alone can recognize antigens and is therefore also known as a single-domain antibody (sdAb). Single-domain antibodies lack light chains and consist only of the heavy chain variable region. Due to their small molecular size, they are also referred to as nanobodies (Nb). With a molecular weight of approximately 13-15 KDa, a diameter of about 2.5 nm, and a length of 4 nm, single-domain antibodies are the smallest antibody fragments with antigen-binding capabilities discovered to date. Their antigen-binding ability and stability are generally comparable to or exhibit higher specificity and antigen affinity than those of full antibodies. Compared to traditional antibodies, single-domain antibodies possess numerous unique properties, such as good stability, the ability to reach special antigenic epitopes, arbitrary combination in a modular fashion, and low production costs. The conventional method for obtaining single-domain antibodies involves multiple steps, comprising repeated immunization of camelids, isolation of B lymphocytes, amplification of the VHH region, construction and screening of display libraries. With the development of synthetic biology, it has become possible to construct high-quality, randomized, large-capacity single-domain antibody libraries based on full synthesis. Currently, VHH single-domain antibodies are widely used in miniaturized genetic engineering antibody research, new drug development, and disease diagnosis and treatment due to their structural stability, small molecular size, good solubility, tolerance to various harsh environments, good formulation stability, ease of recombinant expression, and ease of humanization. The research and development of single-domain antibodies hold great promise in the fields of pharmaceutical uses and clinical diagnosis, and the miniaturization of antibodies is of significant importance for the development of antibody drugs.

### SUMMARY

One of the objectives of the present disclosure is to provide a single-domain antibody targeting the CLL1 antigen and its use in tumor-targeted therapy.

In order to achieve the aforementioned objective, firstly, the present disclosure provides a single-domain antibody named CLL1-VHH-1. This single-domain antibody is composed of a heavy chain variable region (VHH), which may comprise the complementarity-determining region CDR1 located at positions 26-35 of SEQ ID No.1, the complementarity-determining region CDR2 located at positions 50-59 of SEQ ID No.1, and the complementarity-determining region CDR3 located at positions 99-118 of SEQ ID No.1.

The aforementioned single-domain antibody can be a single-domain antibody that specifically binds to the CLL1 protein (anti-CLL1 single-domain antibody).

Furthermore, the amino acid sequence of the heavy chain variable region may be SEQ ID No.1 or an amino acid sequence that shares at least 80% identity with SEQ ID No.1 and possesses the same function.

The single-domain antibody (anti-CLL1 single-domain antibody) consists solely of the variable region (VHH) of the heavy chain antibody, which is sequentially composed of framework region FR1, complementarity-determining region CDR1, framework region FR2, complementarity-determining region CDR2, framework region FR3, complementarity-determining region CDR3, and framework region FR4.

The amino acid sequence of the single-domain antibody CLL1-VHH-1 may be SEQ ID No.1. Specifically, positions 1-25 of SEQ ID No.1 represent framework region FR1, positions 26-35 represent complementarity-determining region CDR1, positions 36-49 represent framework region FR2, positions 50-59 represent complementarity-determining region CDR2, positions 60-98 represent framework region FR3, positions 99-118 represent complementarity-determining region CDR3, and positions 119-129 represent framework region FR4. The nucleotide sequence of the nucleic acid molecule (CLL1-VHH-1 gene) encoding the single-domain antibody CLL1-VHH-1 is shown in SEQ ID No.2.

Among them, positions 1-75 of SEQ ID No.2 represent the coding sequence of framework region FR1, positions 76-105 represent the coding sequence of complementarity-determining region CDR1, positions 106-147 represent the coding sequence of framework region FR2, positions 148-177 represent the coding sequence of complementarity-determining region CDR2, positions 178-294 represent the coding sequence of framework region FR3, positions 295-354 represent the coding sequence of complementarity-determining region CDR3, and positions 355-387 represent the coding sequence of framework region FR4.

The sequences of the complementarity-determining regions are defined according to the Kabat numbering system.

Other variants of the single-domain antibody sequence of the present disclosure, which exhibit improved affinity and/or titer, can be obtained by employing methods known in the art and are comprised within the scope of protection of the present disclosure. For instance, amino acid substitutions can be utilized to obtain antibodies with further enhanced affinity. Alternatively, codon optimization of nucleotide sequences can also be employed to improve translational efficiency in expression systems used for antibody production. Additionally, polynucleotides comprising sequences optimized for antibody specificity through directed evolution methods applied to any nucleic acid sequence of the present disclosure also fall within the scope of the disclosure.

In certain embodiments, substitutions, insertions, or deletions may occur in one or more complementarity-determining regions or framework regions of the single-domain antibody described in the present disclosure, provided that such changes do not substantially reduce the antibody's ability to bind to the antigen. For example, conservative changes (e.g., conservative substitutions, which are well-known to those skilled in the art as not altering the properties and functions of a protein) can be made to the complementarity-determining regions and/or framework regions without substantially reducing binding affinity. Such changes may, for instance, occur outside of the antigen-contacting residues within the complementarity-determining regions.

The present disclosure also provides biological materials related to the single-domain antibody CLL1-VHH-1, which may be any one of the following C1) to C4):

C1) a nucleic acid molecule encoding the heavy chain variable region of the single-domain antibody CLL1-VHH-1;
C2) an expression cassette comprising the nucleic acid molecule described in C1);
C3) a recombinant vector comprising the nucleic acid molecule described in C1) or a recombinant vector comprising the expression cassette described in C2);
C4) a recombinant host cell comprising the nucleic acid molecule described in C1), a recombinant host cell comprising the expression cassette described in C2), or a recombinant host cell comprising the recombinant vector described in C3).

Among them, the expression cassette described in C2), the recombinant vector described in C3), and the recombinant host cell described in C4) are all capable of expressing the single-domain antibody CLL1-VHH-1.

In the aforementioned biological materials, the nucleic acid molecule may be any one of the following:

D1) a DNA molecule with a nucleotide sequence or coding sequence of SEQ ID No.2;
D2) a DNA molecule that shares 75% or more identity with the nucleotide sequence defined in D1) and possesses the same function.

The DNA molecule shown in SEQ ID No.2 encodes the single-domain antibody CLL1-VHH-1 with the amino acid sequence of SEQ ID No.1.

The aforementioned identity of 75% or more may refer to identity of 80%, 85%, 90%, or 95% or more.

Identity of more than 80% may refer to at least 80%, 81%, 82%, 83%, 84%, 85%, 86%, 87%, 88%, 89%, 90%, 91%, 92%, 93%, 94%, 95%, 96%, 97%, 98%, or 99% identity. Identity of more than 85% may refer to at least 85%, 86%, 87%, 88%, 89%, 90%, 91%, 92%, 93%, 94%, 95%, 96%, 97%, 98%, or 99% identity. Identity of more than 90% may refer to at least 90%, 91%, 92%, 93%, 94%, 95%, 96%, 97%, 98%, or 99% identity. Identity of more than 95% may refer to at least 95%, 96%, 97%, 98%, or 99% identity.

In this text, identity refers to the identity of amino acid sequences or nucleotide sequences. The identity of amino acid sequences can be determined using homology search websites on the Internet, such as the BLAST webpage on the NCBI homepage. For example, in Advanced BLAST 2.1, by using blastp as the program, setting the Expect value to 10, turning all Filters OFF, using BLOSUM62 as the Matrix, and setting the Gap existence cost, Per residue gap cost, and Lambda ratio to 11, 1, and 0.85 (default values), respectively, and conducting a search, the identity of amino acid sequences can be calculated, and then the identity value (%) can be obtained.

The vectors described in this text refer to those capable of transporting exogenous DNA or target genes into host cells for amplification and expression. These vectors can be cloning vectors or expression vectors, comprising but not limited to plasmids, phages (such as λ phage or M13 filamentous phage), cosmid (i.e., cosmid plasmid), artificial chromosomes (such as yeast artificial chromosomes (YACs), bacterial artificial chromosomes (BACs), P1 artificial chromosomes (PACs), or Ti plasmid artificial chromosomes (TACs)), viral vectors (such as baculovirus vectors, retroviruses (comprising lentiviruses), adenoviruses, adeno-associated viruses, or herpesviruses (such as herpes simplex virus)), etc. In one or more embodiments of the present disclosure, the vectors are the pComb3XSS plasmid and/or the pcDNA3.1 vector.

The host cells (also referred to as recipient cells) described in this text refer to any type of cells that can be used for introducing vectors. Host cells can be eukaryotic cells (e.g., plant cells, animal cells, fungi, or algae) or prokaryotic cells (e.g., bacteria or protozoa). The host cells are understood to refer not only to specific recipient cells but also to the progeny of such cells, which, due to natural, accidental, or intentional mutations and/or alterations, may not be identical to the original parental cells but are still comprised within the scope of host cells. Suitable host cells are known in the art, comprising but not limited to: plant cells such as *Arabidopsis thaliana, Nicotiana tabacum, Zea mays, Oryza sativa,* and *Triticum aestivum*; animal cells such as mammalian cells (e.g., Chinese hamster ovary cells (CHO cells), African green monkey kidney cells (Vero cells), baby hamster kidney cells (BHK cells), mouse mammary tumor cells (C127 cells), human embryonic kidney cells (HEK293 cells), human HeLa cells, fibroblasts, bone marrow cell lines, T cells, or NK cells), avian cells (e.g., chicken or duck cells), amphibian cells (e.g., *Xenopus laevis* cells or *Andrias davidianus* cells), fish cells (e.g., grass carp, carp, rainbow trout, or catfish cells), insect cells (e.g., Sf21 cells or Sf-9 cells), etc.; fungi such as yeasts from the *Saccharomyces* genus (e.g., *Saccharomyces cerevisiae), Kluyveromyces* genus (e.g., *Kluyveromyces lactis), Pichia* genus (e.g., *Pichia pastoris), Schizosaccharomyces* genus (e.g., *Schizosaccharomyces pombe), Hansenula* genus (e.g., *Hansenula polymorpha),* etc., as well as fungi from the *Fusarium* sp., *Rhizoctonia* sp., *Verticillium* sp., *Penicillium* sp., *Aspergillus* sp., *Cephalosporium* sp., etc.; algae from the *Fucus* sp., *Achnanthes* sp., *Amphiprora* sp., *Amphora* sp., *Ankistrodesmus* sp., *Asteromonas* sp., *Boekelovia* sp., etc.; bacteria from the *Escherichia* sp., *Erwinia* sp., *Agrobacterium* sp., *Flavobacterium* sp., *Alcaligenes* sp., *Pseudomonas* sp., *Bacillus* sp., etc., such as *Escherichia coli, Bacillus subtilis,* or *Bacillus pumilus.* In one or more embodiments of the present disclosure, the host cells are *Escherichia coli* TG1 and/or HEK293 cells.

The recombinant vector described in this text refers to a recombinant DNA molecule constructed by ligating an exogenous target gene with a vector in vitro. It can be constructed in any suitable manner, as long as the constructed recombinant vector can carry the exogenous target gene into the recipient cell and provide the ability for replication, integration, amplification, and/or expression of the exogenous target gene in the recipient cell. In one or more embodiments of the present disclosure, the recombinant vector is pcDNA3.1-CLL1-VHH-1-hFc.

The recombinant vector pcDNA3.1-CLL1-VHH-1-hFc is constructed by adding the human IgG1 Fc gene (hFc gene, with the nucleotide sequence shown as SEQ ID No. 3) to the 3' end of the coding gene (SEQ ID No. 2) of the single-domain antibody CLL1-VHH-1 for ease of purification, resulting in the CLL1-VHH-1-hFc fusion gene. Subsequently, a *BamHI* restriction site is added before the start codon at the 5' end of the fusion gene, and an *EcoRI* restriction site is added after the stop codon at the 3' end. The fusion gene is then cloned between the *BamHI* and *EcoRI* recognition sites of the eukaryotic expression vector pcDNA3.1, yielding the recombinant eukaryotic expression vector pcDNA3.1-CLL1-VHH-1-hFc, which expresses the single-domain antibody CLL1-VHH-1 fused with hFc at the C-terminus (SEQ ID No. 4). The recombinant eukaryotic expression vector pcDNA3.1-CLL1-VHH-1-hFc comprises the coding gene (SEQ ID No. 2) of the single-domain antibody CLL1-VHH-1.

The nucleotide sequence of the human IgG1 Fc gene (696 bp) is shown as SEQ ID No. 3, and its encoded amino acid sequence is shown as SEQ ID No. 4.

The recombinant host cells described in this text refer to recombinant host cells obtained by manipulating and modifying the genes of target host cells, resulting in altered functions. These comprise recombinant host cells obtained by introducing an exogenous target gene or recombinant vector into the target host cells, or recombinant host cells obtained by directly performing gene editing on the endogenous genes of the target host cells. The recombinant host cells are understood to refer not only to specific recombinant host cells but also to the progeny of such cells, which, due to natural, accidental, or intentional mutations and/or alterations, may not be identical to the original parental cells but are still comprised within the scope of recombinant host cells. In one or more embodiments of the present disclosure, the recombinant host cells are obtained by introducing the recombinant vector pcDNA3.1-CLL1-VHH-1-hFc into HEK293 cells.

The present disclosure also provides any one of the following uses of the single-domain antibody CLL1-VHH-1 or the biological materials mentioned above:

E1) a use in the preparation of a drug for preventing or treating tumors (comprising tumors expressing the CLL1 antigen), or use in preventing or treating tumors;

E2) a use in the preparation of a drug for preventing or treating diseases related to the CLL1 target, or use in preventing or treating diseases related to the CLL1 target;

E3) a use in the preparation of products for screening, diagnosing, or aiding in the diagnosis of diseases related to the CLL1 target, or use in screening, diagnosing, or aiding in the diagnosis of diseases related to the CLL1 target;

E4) a use in detecting the CLL1 protein or in the preparation of products for detecting the CLL1 protein;

E5) a use in the preparation of products for binding to the CLL1 protein;

E6) a use in mediating drugs to specifically recognize tumors expressing the CLL1 antigen or in the preparation of products that mediate drugs to specifically recognize tumors expressing the CLL1 antigen;

E7) a use in in vivo imaging of the CLL1 protein or in the preparation of products for in vivo imaging of the CLL1 protein;

E8) a use in the preparation of products targeting the CLL1.

In this context, the CLL1 target-related diseases can be CLL1-positive cancers (such as leukemia).

In this context, the CLL1-positive cancers mentioned herein can comprise, but are not limited to, Acute Myeloid Leukemia (AML), Myelodysplastic Syndromes (MDS), and Chronic Myeloid Leukemia (CML, also known as chronic granulocytic leukemia).

The product described in E6) that mediates the specific recognition of tumors expressing the CLL1 antigen by the drug can be a drug delivery system specifically targeting the CLL1 protein, which comprises the single-domain antibody CLL1-VHH-1.

Furthermore, the drug delivery system also comprises therapeutically active drugs (therapeutic drugs) or diagnostic agents that are coupled or bound to the single-domain antibody CLL1-VHH-1.

Moreover, the therapeutic drugs comprise, but are not limited to, chemotherapy drugs, anti-tumor drugs, antiviral drugs, drugs for autoimmune diseases, and drugs for anti-infectious diseases.

The anti-tumor drugs may comprise chemical drugs or cytotoxins.

The cytotoxins can be tubulin inhibitors (such as maytansine and auristatin), DNA synthesis inhibitors (such as calicheamicin), or RNA synthesis inhibitors (such as amanitin).

Furthermore, the therapeutic drugs can be those used for treating CLL1 target-related diseases.

Additionally, the therapeutic drugs can be one or more of monoclonal antibodies or their binding fragments, proteins, peptides, RNA molecules, DNA molecules, siRNA molecules, RNAi molecules, ssRNA molecules, growth factors, enzyme inhibitors, and binding proteins.

Furthermore, the diagnostic agents can be selected from one or more of imaging agents, contrast agents, fluorescent labels, radioactive labels, magnetic resonance imaging labels, and spin labels.

The imaging agents can be selected from one or more of radionuclides, biotin, fluorophores, antibodies, horseradish peroxidase, alkaline phosphatase, nanoparticles, quantum dots, detectable nanodroplets of anticancer agents, liposomal drugs, and cytokines.

The drug delivery system can be a liposomal drug delivery system, polymeric micelle drug delivery system, polymeric disc drug delivery system, or nanoparticle drug delivery system.

The drug delivery system can be used for targeted drug transport and/or site-specific drug release.

The product described in E8) can be immune cells.

Furthermore, the immune cells comprise, but are not limited to, CAR-modified cells (CAR cells), TCR cells, TIL cells, CIK cells, CTL cells, T cells, NK cells, NKT cells, $\gamma\delta$ T cells, or macrophages.

Furthermore, the CAR-modified cells (CAR cells) contain or express a chimeric antigen receptor (CAR), which may contain the single-domain antibody CLL1-VHH-1.

Furthermore, the TCR cells, TIL cells, CIK cells, CTL cells, T cells, NK cells, NKT cells, $\gamma\delta$ T cells, or macrophages contain or express the single-domain antibody CLL1-VHH-1.

Furthermore, the antigen recognition domain of the chimeric antigen receptor can be the single-domain antibody CLL1-VHH-1.

Furthermore, the CAR cells comprise, but are not limited to, CAR-T cells, CAR-NK cells, CAR-macrophages (CAR-M cells), CAR-iPSCs, or CAR-PSCs.

Furthermore, the TCR cells comprise, but are not limited to, TCR-T cells or TCR-NK cells.

The detection of the CLL1 protein described herein can involve determining whether a test sample comprises the CLL1 protein and/or measuring the content of the CLL1 protein in the test sample.

The test sample can be a cellular or tissue sample.

The products used for detecting the CLL1 protein comprise those that utilize methods such as enzyme-linked immunosorbent assay (ELISA), immunofluorescence assay, radioimmunoassay, luminescent immunoassay, colloidal gold immunochromatography, agglutination assay, or immunoturbidimetry to detect antigen-antibody binding.

The products described herein can be reagents, reagent kits, chips, or test strips.

The present disclosure also provides a pharmaceutical composition that may contain the single-domain antibody CLL1-VHH-1 or chimeric antigen receptor (CAR)-modified cells, wherein the chimeric antigen receptor (CAR) may contain the single-domain antibody CLL1-VHH-1.

Furthermore, the antigen recognition domain of the chimeric antigen receptor can be the single-domain antibody CLL1-VHH-1.

The cells can be T cells, NK cells, $\gamma\delta$ T cells, NKT cells, macrophages, or stem cells.

The CAR-modified cells (CAR cells) can be CAR-T cells, CAR-NK cells, CAR-macrophages (CAR-M cells), CAR-iPSCs, or CAR-PSCs, but are not limited thereto.

Furthermore, the pharmaceutical composition may also contain a pharmaceutically acceptable carrier.

The pharmaceutically acceptable carrier can be a diluent, excipient, filler, binder, wetting agent, disintegrant, absorption enhancer, adsorbent carrier, surfactant, or lubricant.

The pharmaceutical composition has at least one of the following uses:

F1) for preventing or treating tumors;
F2) for preventing or treating diseases related to the CLL1 target;
F3) for mediating the specific recognition of tumors expressing the CLL1 antigen by drugs.

The present disclosure also provides reagents or reagent kits that may contain the single-domain antibody CLL1-VHH-1, with at least one of the following uses:

G1) detecting the CLL1 protein;
G2) screening, diagnosing, or aiding in the diagnosis of diseases related to the CLL1 target;
G3) for in vivo imaging of the CLL1 protein.

The reagent kits can be chemiluminescent immunoassay kits, enzyme-linked immunosorbent assay kits, colloidal gold immunoassay kits, or fluorescence immunoassay kits, but are not limited thereto.

The present disclosure also provides a method for preparing the single-domain antibody CLL1-VHH-1, which may comprise the following steps: constructing a recombinant expression vector comprising a nucleic acid molecule encoding the variable region of the heavy chain of the single-domain antibody CLL1-VHH-1; introducing the recombinant expression vector into host cells to obtain recombinant cells expressing the single-domain antibody; culturing the recombinant cells and obtaining the single-domain antibody through isolation and purification.

Furthermore, the nucleic acid molecule encoding the variable region of the heavy chain of the single-domain antibody CLL1-VHH-1 may be any of the following:

H1) a DNA molecule with the nucleotide sequence or coding sequence being SEQ ID No.2;
H2) a DNA molecule that shares 75% or more identity with the nucleotide sequence defined in H1) and has the same function.

Furthermore, the host cells may be HEK293 cells.

Furthermore, the introduction may involve transforming host cells with a vector carrying the gene of the single-domain antibody of the present disclosure using any known transfection method, such as calcium phosphate co-precipitation, liposome-mediated transfection, electroporation, or viral vector methods.

The present disclosure also provides a method for diagnosing or aiding in the diagnosis of diseases related to the CLL1 target, which may comprise the following steps: obtaining a sample from a subject, then detecting the content of the CLL1 protein in the sample using the single-domain antibody CLL1-VHH-1, and performing diagnosis or aiding in the diagnosis of diseases related to the CLL1 target based on the content of the CLL1 protein (compared with the CLL1 protein content in disease diagnostic criteria).

The present disclosure also provides a method for screening diseases related to the CLL1 target, which may comprise the following steps: obtaining a sample from a subject, then detecting the content of the CLL1 protein in the sample using the single-domain antibody CLL1-VHH-1, and performing screening for diseases related to the CLL1 target based on the content of the CLL1 protein (compared with the CLL1 protein content in disease screening criteria).

The present disclosure also provides a method for preventing or treating diseases related to the CLL1 target, which may comprise administering the single-domain antibody CLL1-VHH-1 or the pharmaceutical composition described herein to a subject suffering from diseases related to the CLL1 target.

In the above methods, the diseases related to the CLL1 target may be tumors related to the CLL1 target.

In the above methods, the tumors related to the CLL1 target may be CLL1-positive cancers.

In the above methods, the CLL1-positive cancers may be acute myeloid leukemia, myelodysplastic syndromes, or chronic myeloid leukemia.

The present disclosure also provides an in vitro method for detecting the CLL1 protein, which may comprise detecting the CLL1 protein using the single-domain antibody CLL1-VHH-1 or the reagents or reagent kits described herein.

The present disclosure also provides an antibody-drug conjugate comprising an antibody moiety and a conjugated moiety, wherein the antibody moiety may comprise the single-domain antibody CLL1-VHH-1.

Furthermore, the conjugated moiety may comprise detectable labels, chemotherapeutic agents, or cytotoxins.

The antibody moiety and the conjugated moiety may be covalently linked directly or via a linker.

The detectable labels may be selected from enzymes (such as horseradish peroxidase or alkaline phosphatase), chemiluminescent reagents (such as acridinium ester compounds, luminol and its derivatives, or ruthenium derivatives), fluorescent dyes (such as fluorescein or fluorescent proteins), radionuclides, or biotin.

The chemotherapeutic agents may be drugs capable of killing tumor cells.

The cytotoxins may be tubulin inhibitors (such as maytansinoids or auristatins), DNA synthesis inhibitors (such as calicheamicin), or RNA synthesis inhibitors (such as amanitin).

The purposes of the uses and methods described in the present disclosure may comprise disease diagnosis, disease prognosis, and/or disease treatment, or they may serve non-disease diagnostic, non-disease prognostic, and/or non-disease therapeutic purposes; their direct objectives may involve obtaining information as intermediate results for disease diagnosis, disease prognosis, and/or disease treatment, or they may have non-disease diagnostic, non-disease prognostic, and/or non-disease therapeutic objectives.

CLL1, referred to herein as C-type lectin-like molecule 1 (also known as C-type lectin domain family 12 member A, CLEC12A), is a type II transmembrane glycoprotein that plays an important role in immune regulation as an inhibitory receptor. CLL1 is present in myeloid cells in peripheral blood and bone marrow, as well as in most acute myeloid leukemia (AML) cells. It is also expressed on $CD34^+CD38^-$ stem cells in most AML cases, whereas normal $CD34^+CD38^-$ stem cells do not express CLL1. Due to its unique expression pattern, CLL1 has emerged as a potential target for AML treatment and diagnosis. CLL1 is also expressed in myelodysplastic syndromes (MDS) and chronic myeloid leukemia (CML) cells.

In this disclosure, alpacas were immunized with CLL1 recombinant protein to obtain single-domain antibody genes, and a single-domain antibody expression library was constructed. High-affinity anti-CLL1 single-domain antibodies (CLL1-VHH-1) were then screened from the library using phage display technology. After sequencing the antibody gene sequences, CLL1-VHH-1 was prepared using genetic engineering methods. Further experiments were conducted to evaluate the affinity and specificity of the obtained antibody, as well as to detect the antitumor activity mediated by the single-domain antibody CLL1-VHH-1, comprising measurements of NK cell effector function and antibody-dependent cellular cytotoxicity (ADCC) activity. The experimental results demonstrated that the single-domain antibody (CLL1-VHH-1) obtained through alpaca immunization and screening exhibited an affinity constant of 48.3 nM for K562-CLL1 cells, indicating a high affinity for the CLL1 protein. Additionally, the single-domain antibody CLL1-VHH-1 of the present disclosure showed good specificity and effectively targeted CLL1-positive cells (cells expressing the CLL1 protein, CLL1$^+$ cells). The antibody CLL1-VHH-1 of this disclosure more effectively promoted NK cell secretion of IFN$\gamma$, activated NK cell effector function, enhanced NK cell efficacy against CLL1$^+$ target cells, thereby improving antitumor activity, and significantly induced NK cell cytotoxicity (ADCC), demonstrating strong specific killing ability against CLL1-positive cells.

The single-domain antibody of the present disclosure can be expressed and produced in prokaryotic cells, yeast cells, eukaryotic cells, and any recombinant system. It can be formulated into specific antibody drugs for the clinical prevention and treatment of diseases related to the CLL1 target (such as acute myeloid leukemia, myelodysplastic syndromes, or chronic myeloid leukemia). It can also be used to prepare CAR cells targeting CLL1 or detection kits for the CLL1 protein. The single-domain antibody drug of the present disclosure is structurally stable, has a small molecular size, is easy to recombinantly express, has low production costs, can be used alone or as a drug delivery system to carry relevant drugs, and holds great promise and significance in drug uses and clinical diagnostics.

**BRIEF DESCRIPTION OF THE DRAWINGS**

FIG. 1 shows the agarose gel electrophoresis results of total RNA extracted from alpaca PBMCs.

FIG. 2 presents the agarose gel electrophoresis results of colony PCR for the bacterial library.

FIG. 3 illustrates the results of detecting CLL1 expression on different tumor cell lines using the FITC-labeled CLL1-VHH-1 antibody. Here, "Isotype" represents the CLL1-VHH-1-hFc antibody not labeled with FITC, while "CLL1" represents the FITC-CLL1-VHH-1-hFc antibody.

FIG. 4 displays the results of measuring the effector function of NK cells mediated by the CLL1-VHH-1 antibody in Example 3. Here, "VHH-hFc" refers to the CLL1-VHH-1 antibody.

FIG. 5 shows the results of measuring the ADCC activity induced by the CLL1-VHH-1 antibody in Example 3.

**DETAILED DESCRIPTION**

The present disclosure will be further described in detail below in conjunction with specific embodiments. The examples provided are solely for the purpose of illustrating the disclosure and are not intended to limit its scope. The examples provided hereinafter can serve as a guide for further improvements by those skilled in the art and do not in any way constitute a limitation on the present disclosure.

Unless otherwise specified, the experimental methods in the following examples are conventional methods, carried out in accordance with the techniques or conditions described in the literature within the field or according to the product instructions. Unless otherwise specified, the materials and reagents used in the following examples are commercially available.

The pComb3XSS plasmid used in the following examples is a product of Beijing Zoman Biotechnology Co., Ltd., with the product number ZK129.

The helper phage M13K07 used in the following examples is a product of Chengdu Renyu Biotechnology Co., Ltd., with the product number A001-1.

The K562 cells (CLL1-negative cells) used in the following examples are products of Cobioer Biosciences Co., Ltd., with the product number CBP60529; MM1.S cells (CLL1-negative cells) are products of Beijing Biobw Biotech Co., Ltd., with the product number bio-129535; U937 cells (CLL1-positive cells) are products of Beijing Biobw Biotech Co., Ltd., with the product number bio-73180; U266 cells (CLL1-negative cells) are products of Beijing Biobw Biotech Co., Ltd., with the product number bio-69276; Jurkat cells (CLL1-negative cells) are products of Beijing Biobw Biotech Co., Ltd., with the product number bio-68218; THP-1 cells (CLL1-positive cells) are products of Beijing Biobw Biotech Co., Ltd., with the product number bio-68161.

The primary peripheral blood mononuclear cells (PBMCs) used in the following examples are derived from the venous blood of healthy volunteers and are referred to as primary PBMC cells for short.

The preparation method of CLL1-positive K562-CLL1 cells in the following examples involves inserting the CLL1 gene between the *BamHI* and *EcoRI* sites of pcDNA3.1, transfecting the prepared recombinant vector into K562 cells to obtain CLL1-positive K562-CLL1 cells.

The vector pcDNA3.1 used in the following examples is a product of Changsha Abiowell Biotechnology Co., Ltd., with the product number HG-VPI0001.

Some sequences in the following examples are shown below:

Nucleotide sequence of the human IgG1Fc gene (696 bp):

gagcccaaatctgctgacaaaactcacacatgcccaccgtgcccagcacctgaactcctggggggaccgtcagtcttcctc ttcccccaaaacccaaggacaccctcatgatctcccggacccccgaggtcacatgcgtggtggtggacgtgagccacgaagac cctgaggtcaagttcaactggtacgtggacggcgtggaggtgcataatgccaagacaaagccgcgggaggagcagtacaacag cacgtaccgtgtggtcagcgtcctcaccgtcctgcaccaggactggctgaatggcaaggagtacaagtgcaaggtctccaacaaa gccctcccagcccccatcgagaaaaccatctccaaagccaaagggcagccccgagaaccacaggtgtacaccctgcccccatc ccgggaggagatgaccaagaaccaggtcagcctgacctgcctggtcaaaggcttctatcccagcgacatcgccgtggagtgggga gagcaatgggcagccggagaacaactacaagaccacgcctcccgtgctggactccgacggctccttcttcctctacagcaagctc accgtggacaagagcaggtggcagcaggggaacgtcttctcatgctccgtgatgcatgaggctctgcacaaccactacacgcag aagagcctctccctgtctccgggtaaa (SEQ ID No.3)

Amino acid sequence of human IgG1Fc (232 aa):

EPKSADKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCK VSNKALPAPIEKTISKAKGQPREPQVYTLPPSREEMTKNQVSLTCLVKGFYPSDIAVE WESNGQPENNYKTTPPVLDSDGSFFLYSKLTVDKSRWQQGNVFSCSVMHEALHNH YTQKSLSLSPGK (SEQ ID No.4)

Example 1: Preparation of a Single-Domain Antibody Specifically Binding to the CLL1 Protein

In this example, alpacas were immunized with the CLL1 recombinant protein to obtain single-domain antibody genes. A single-domain antibody expression library was constructed, and then high-affinity single-domain antibodies specifically binding to the CLL1 protein (referred to as anti-CLL1 single-domain antibodies for short) were screened from the library using phage display screening technology.

1. Animal Immunization

1.1 Acquisition of Antigens

The CLL1 recombinant proteins used as antigens are as follows:

CLL1 immunization antigen: The CLL1-hIgG1Fc (B918601) protein (derived from HEK293 cells) is a product of Shanghai Bio-Antibody Biotech Co., Ltd.

CLL1 screening antigen: The CLL1-6×His (B918602) protein (derived from HEK293 cells) is a product of Shanghai Bio-Antibody Biotech Co., Ltd.

1.2 Immunization of Alpacas

Alpacas (2-3 years old, weighing approximately 50 kg) were immunized four times with the CLL1 immunization antigen CLL1-hIgG1Fc. Each time, 0.5 mg of CLL1-hIgG1Fc was injected subcutaneously, with a 2-week interval between two immunizations. Before the next immunization, 5 ml of blood was drawn, and the plasma was separated to detect the antibody titer after the previous immunization. The titer after the fourth immunization was detected one week after the injection.

The titer of antibodies in the serum was detected by the indirect ELISA method, and the steps are as follows.

(1) The immunization antigen CLL1-hIgG1Fc was diluted to 2 $\mu$g/mL with PBS and coated onto enzyme-linked immunosorbent assay (ELISA) plates at 100 $\mu$L per well, followed by incubation overnight at 4°C. The control wells were coated with 3% bovine serum albumin (BSA).

(2) The antigen solution was discarded, and the ELISA plates were washed three times with 200 µL of PBST solution (1×PBS solution comprising 0.05% Tween 20), with each wash lasting for 5 minutes.

(3) The ELISA plates were blocked with 200 µL of blocking solution PBSTB (PBST comprising 2% BSA) and incubated at room temperature for 1.5 hours.

(4) The blocking solution was discarded, and the ELISA plates were washed three times with 200 µL of PBST solution, with each wash lasting for 5 minutes.

(5) The serum to be tested for titer was gradient-diluted with the blocking solution at ratios of 1:10, 1:100, 1:1000, 1:10000, 1:100000, 1:1000000, and 1:10000000. Then, 100 µL of the diluted serum was added to each well of the ELISA plates, and the plates were incubated at room temperature for 2 hours.

(6) The serum was discarded, and the ELISA plates were washed four times with 200 µL of PBST solution, with each wash lasting for 5 minutes.

(7) Goat anti-llama antibody (Goat Anti-Llama IgG H&L (HRP), Abcam, ab112786) diluted 1:10000 with the blocking solution was added to each well at 100 µL per well, and the plates were incubated at room temperature for 1 hour.

(8) The detection antibody was discarded, and the ELISA plates were washed four times with 200 µL of PBST solution, with each wash lasting for 5 minutes.

(9) 100 µL of TMB substrate solution was added to each well, and the plates were allowed to develop color for 2-3 minutes.

(10) 100 µL of reaction stop solution was added to each well.

(11) The absorbance at 450 nm was measured using a microplate reader within 30 minutes. The criterion for determining positive wells: The $OD_{450}$ reading of the immunized serum sample was greater than that of the non-immunized serum, and the reading was greater than 0.5.

1.3 Immunization Results

The results of serum titer detection are shown in Table 1. The values in bold indicate positive wells. After four immunizations, the serum titer in the alpaca reached $10^5$, indicating that the antibody level reached $10^5$.

Table 1: Results of Serum Titer Detection

| different antibody dilutions | The antigen coating | | | | |
|---|---|---|---|---|---|
| | non-imm unized | The first immunization | The second immunization | The third immunization | The fourth immunization |
| 1:10 | 0.1497 | **1.5622** | **1.4494** | **1.5722** | **1.3833** |
| 1:100 | 0.0371 | **1.2325** | **2.0392** | **2.0212** | **1.9837** |
| 1:1000 | 0.0265 | 0.3245 | **2.0358** | **2.0520** | **2.1111** |
| 1:10000 | 0.0325 | 0.1251 | **1.6479** | **1.8318** | **1.8690** |
| 1:100000 | 0.0266 | 0.0911 | **0.7545** | **0.9391** | **0.9838** |
| 1:1000000 | 0.0205 | 0.0585 | 0.1141 | 0.1692 | 0.1929 |
| 1:10000000 | 0.0061 | 0.0172 | 0.0239 | 0.0882 | 0.0714 |
| PBSTB | 0.0000 | 0.0000 | 0.0000 | 0.0000 | 0.0000 |

As shown in Table 1: After the fourth immunization, the antibody level in the alpaca reached $10^5$. The antigen coating refers to coating with the immunizing antigen CLL1-hIgG1Fc. PBSTB is the blocking solution.

2. Construction of the Phage Library

After successful immunization, peripheral blood mononuclear cells (PBMCs) from the alpaca were collected to construct a phage library, namely, the anti-CLL1 VHH phage antibody library.

2.1 RNA Extraction and Reverse Transcription

Alpaca PBMCs were isolated using density gradient centrifugation and stored in Trizol. Total RNA was extracted from the alpaca PBMCs, and the obtained total RNA was reverse-transcribed into cDNA using a Takara reverse transcription kit. The specific steps are as follows:

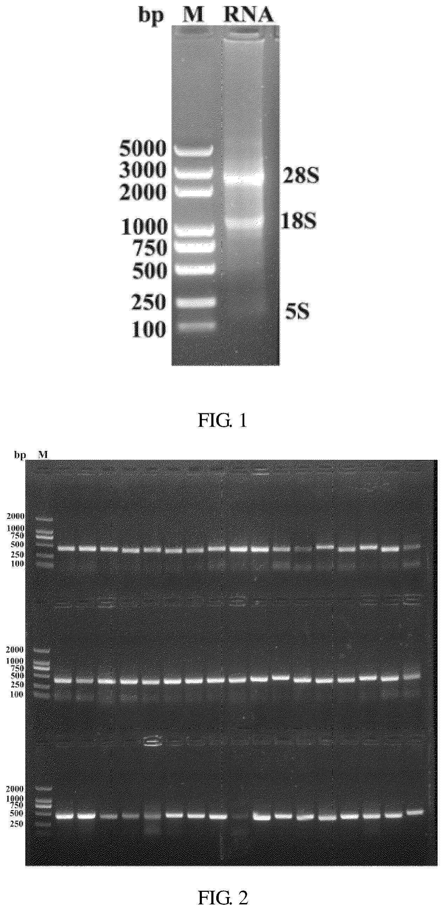

(1) transferring the alpaca PBMCs stored in Trizol to a 1.5 mL centrifuge tube, adding one-fifth volume of chloroform, mixing well, and letting stand at room temperature for 5 minutes;
(2) centrifuging at 12,000 g at 4°C for 15 minutes;
(3) transferring the supernatant to a new centrifuge tube, adding an equal volume of isopropanol, and letting stand at room temperature for 10 minutes;
(4) centrifuging at 12,000 g at 4°C for 10 minutes;
(5) washing the precipitate with 1 mL of 75% ethanol, centrifuging at 7,500 g for 5 minutes to remove the ethanol, and then drying the precipitate;
(6) dissolving the precipitate in an appropriate amount of RNase-free water to obtain the extracted total RNA; a total of 181.2 $\mu$g of total RNA was obtained (FIG. 1);
(7) reverse-transcribing the total RNA using the Takara reverse transcription kit: the total RNA sample was divided into two portions; one portion used the Oligo dT Primer from the kit as the primer, and the other portion used the Random 6-mers from the kit as the primer; the total RNA was reverse-transcribed into cDNA according to the operating instructions of the kit, and a total of 31.7 $\mu$g of cDNA was obtained.

2.2 PCR Amplification of the VHH Target Fragment of the CLL1 Antibody

Using 8 ng of cDNA as the template, the VHH target fragment was amplified by conventional PCR, and a total of 83 $\mu$g was obtained.

2.3 Enzymatic Digestion and Ligation

1.Using pComb3XSS as the phage plasmid vector, digesting the pComb3XSS vector and the PCR product of VHH (i.e., the VHH target fragment) with the restriction endonucleases *Spe*I and *Sac*I respectively, and incubating at 37°C for 4 hours;

2. purifying the digested pComb3XSS vector and the PCR product of VHH using a DNA recovery and purification kit, and storing it at 4°C; and

3. ligating and recovering the digested pComb3XSS vector and the VHH target fragment; wherein a total of 3.1 $\mu$g of the ligation product was obtained.

2.4 Construction of the Bacterial Library

(1) taking 100 ng of the ligation product and performing electroporation with 50 $\mu$L of competent *Escherichia coli* TG1 cells;
(2) recovering at 37°C for 1 hour, taking 100 $\mu$L of the transformed product, performing 10-fold gradient dilution, and then spreading it on plates, culturing overnight at 37°C;
(3) calculating the number of transformed colonies obtained from all electroporation reactions based on the dilution factor and the number of single colonies; wherein the library capacity of the bacterial library was $2.6 \times 10^8$ colony-forming units (cfu);
(4) randomly selecting several single colonies from the gradient dilution plates for colony PCR, wherein the agarose gel electrophoresis results showed (FIG. 2) that all the selected single colonies were positive clones, with a positive rate of 100%;
(5) spreading all the remaining bacterial solutions (i.e., the gradient dilutions of the transformed products) evenly on 20 15 cm culture plates (2×YT medium comprising 100 $\mu$g/mL ampicillin and 2% agarose), and culturing upside down overnight at 37°C.
(6) scraping off the bacterial colonies on the overnight-cultured plates using 2×YT liquid medium, placing them in a 50 mL centrifuge tube, measuring the $OD_{600} = 120$, adding glycerol to a final concentration of 20%, and storing at -80°C to obtain the bacterial library; and
(7) translating the sequencing sequences of the above positive single-colony clones into protein sequences, wherein sequence diversity comparison showed that they were all independent sequences with good diversity, and the diversity of the bacterial library met the requirements.

2.5 Preparation of the Phage Library

(1) inoculating the bacterial library into 100 mL of 2×YT liquid medium (comprising 100 $\mu$g/mL ampicillin) and culturing at 37°C and 250 rpm until the $OD_{600}$ reaches 0.5-0.55;

(2) adding the helper phage M13K07 at a ratio of bacteria: phages=1:20 and incubating at 37°C and 250 rpm for 30 minutes;

(3) adding kanamycin to a final concentration of 50 $\mu$g/mL and culturing overnight at 30°C and 250 rpm;

(4) centrifuging the overnight-cultured bacterial solution at 4,000 rpm at 4°C for 20 minutes, and transfering the supernatant to a new 50 mL centrifuge tube;

(5) adding one-fourth volume of 4°C pre-cooled 20% PEG/2.5 M NaCl solution, mixing well, and incubating on ice for at least 30 minutes;

(6) centrifuging at 4,000 rpm at 4°C for 20 minutes, discarding the supernatant, and inverting for 2 minutes.

(7) adding 1 mL of PBS to resuspend, centrifuging at 12,000 rpm at 4°C for 10 minutes;

(8) transfering the supernatant to a new centrifuge tube, adding one-fourth volume of 4°C pre-cooled 20% PEG/2.5 M NaCl solution again, mixing well to form a white suspension, and incubating on ice for at least 10 minutes; and

(9) Centrifuging at 12,000 rpm at 4°C for 10 minutes, discarding the supernatant, resuspend with 1 mL of PBS, centrifuging at 12,000 rpm for 10 minutes, transfering the supernatant to a new centrifuge tube, and storing at -80°C, wherein this is the purified phage library, with a titer of $1\times10^{13}$ plaque-forming units per milliliter (pfu/mL).

3. Affinity Screening

Three rounds of affinity screening were conducted on the phage library using the screening antigen (CLL1-6×His), which comprised titer determination, amplification of the phage eluate, and phage purification. After the three rounds of affinity screening, when the enrichment level of anti-CLL1 VHH positive antibodies in the screened library reached the standard, the next step of positive monoclonal antibody screening could be carried out. The specific steps are as follows.

3.1 First-Round Screening

3.1.1 First-Round Screening Method

(1) coating the immunotubes with the screening antigen (CLL1-6×His) (50 $\mu$g per tube, with 2 mL of PBS per tube as the coating buffer) and rotating slowly at 4°C overnight, meanwhile, coating BSA (50 $\mu$g per tube, with 2 mL of PBS per tube as the coating buffer) in parallel as a control;

(2) discarding the liquid in the overnight-coated immunotubes, adding 2 mL of PBS buffer, and washing at room temperature three times, rotating for 5 minutes each time;

(3) adding 2 mL of 3% skim milk powder and blocking by rotating at room temperature for 2 hours;

(4) discarding the liquid in the blocked immunotubes, adding 2 mL of PBST buffer, and washing at room temperature three times, rotating for 5 minutes each time;

(5) discarding the washing liquid in the immunotubes, adding the phage library as the input phage library for the first-round screening according to the following formula, adding PBS buffer to 2 mL, and rotating and incubating at room temperature for 1 hour;

$$V(ul) = \frac{10^{12}}{T_{library}} \times 1000$$

wherein, V is the volume of the phage added (in $\mu$L), and $T_{library}$ is the titer of the phage.

(6) discarding the liquid in the immunotubes, add 2 mL of PBST (1×PBS with 0.1% Tween 20, the same below) buffer, and washing the immunotubes 20 times at room temperature, rotating for 5 minutes each time;

(7) discarding the liquid in the immunotubes, adding 1 mL of 0.25 mg/mL Trypsin solution, and eluting by rotating at room temperature for 30 minutes;

(8) adding 10 μL of 10% AEBSF (a serine protease inhibitor) to terminate the elution, wherein the solution in the immunotubes is transferred to a new 1.5 mL centrifuge tube, which is the phage eluate of the first-round screening (referred to as the first-round phage eluate for short).

3.1.2 Initial Phage Titer Determination

(1) streaking the TG1 strain stored at -80°C on a 2×YT solid medium plate for single colony isolation and culturing overnight at 37°C;
(2) picking a single colony from the single-colony plate into 5 mL of 2×YT medium and culturing overnight at 37°C;
(3) transferring 50 μL of the bacterial solution to 5 mL of 2×YT liquid medium and culturing at 37°C and 250 rpm until $OD_{600}$ = 0.5-0.55 to obtain the TG1 bacterial solution;
(4) taking 10 μL of the first-round phage eluate and performing 10-fold gradient dilution in a 1.5 mL centrifuge tube, that is, taking 10 μL of the first-round phage eluate into 90 μL of PBS, then taking 10 μL from it into 90 μL of PBS, and so on, wherein a total of 12 gradients are diluted to $10^{-12}$;
(5) adding 90 μL of the TG1 bacterial solution to each diluted centrifuge tube, mixing by shaking, and incubating at 37°C for 30 minutes;
(6) drop 5 μL of the bacterial solution from each diluted centrifuge tube onto a 2×YT solid medium plate (with Amp) and letting stand for a few minutes, then culturing upside down overnight at 37°C;
(7) counting the number of clearly distinguishable single colonies on the plates with different dilution factors, calculating the number of phagemids per milliliter of the phage solution, i.e., the titer of the phage library, according to the following formula:

$$T\ (pfu/ml)\ = N\ \times D\ \times 400$$

wherein, T is the titer of the phage (in pfu/mL), D is the dilution factor, and N is the number of single colonies on the plate with the corresponding dilution factor.

3.1.3 Amplification of the First-Round Phage Eluate

(1) streaking the TG1 strain stored at -80°C on a 2×YT solid medium plate for single colony isolation and culturing overnight at 37°C;
(2) picking a single colony from the single-colony plate into 5 mL of 2×YT medium and culturing overnight at 37°C;
(3) transferring 50 μL of the bacterial solution to 5 mL of 2×YT liquid medium and culturing at 37°C and 250 rpm until $OD_{600}$ = 0.5-0.55;
(4) adding 500 μL of the first-round phage eluate and continuing to culture at 37°C and 250 rpm for 30 minutes;
(5) spreading all the bacterial solution evenly on three 15 cm round medium plates comprising 100 μg/mL Amp and culturing overnight at 37°C;
(6) add 2×YT liquid medium to the surface of the medium plates, gently scraping off the colonies with a spreader, and collecting the bacterial solution into a 15 mL centrifuge tube to obtain the amplified bacterial sub-library, adding glycerol to a final concentration of 20%, and measuring the $OD_{600}$ value of the bacterial solution using a spectrophotometer to obtain the $OD_{600}$ value of the eluate bacterial library.
(7) transferring the eluate bacterial library to 100 mL of 2×YT liquid medium (comprising 100 μg/mL Amp) according to the following formula with an initial $OD_{600}$ = 0.1:

$$V\ (ul) = \frac{10}{OD600} \times 1000$$

wherein, V is the volume of the transferred bacterial solution (in μL), and $OD_{600}$ is the $OD_{600}$ value of the constructed eluate bacterial library;
(8) culturing at 37°C and 250 rpm until the $OD_{600}$ of the bacterial solution reaches 0.5-0.55;
(9) adding the helper phage M13K07 according to the ratio of bacteria:phages = 1:20 using the following formula:

$$V\ (ml) = \frac{OD600 \times 1.6 \times 10^{11}}{T_{helper-phage}}$$

wherein, V is the volume of the added helper phage (in mL), $T_{helper-phage}$ is the titer of the helper phage, and $OD_{600}$ is the $OD_{600}$ value of the bacterial solution;

(10) continuing to culture at 37°C and 250 rpm for 30 minutes;

(11) adding kanamycin to a final concentration of 50 μg/mL and culturing overnight at 30°C and 200 rpm.

3.1.4 First-Round Phage Purification

(1) transferring the overnight-cultured bacterial solution to a new 50 mL centrifuge tube and centrifuging at 4,000 rpm at 4°C for 20 minutes;

(2) transferring the supernatant to a new 50 mL centrifuge tube, adding one-fourth volume of 4°C pre-cooled 20% PEG/2.5 M NaCl solution, mixing well, and placing on ice for at least 30 minutes;

(3) centrifuging at 4,000 rpm at 4°C for 20 minutes and discarding the supernatant;

(4) adding 1 mL of PBS to resuspend the precipitate, transferring to a new 1.5 mL centrifuge tube, and centrifuging at 13,000 rpm at 4°C for 15 minutes;

(5) transferring the supernatant to a new 1.5 mL centrifuge tube, adding one-fourth volume of 4°C pre-cooled 20% PEG/2.5 M NaCl solution, mixing well, and placing on ice for at least 10 minutes;

(6) centrifuging at 13,000 rpm at 4°C for 10 minutes, discarding the supernatant, and resuspending the precipitate with 1 mL of PBS; and

(7) centrifuging at 13,000 rpm at 4°C for 2 minutes, transferring the supernatant to a new 1.5 mL centrifuge tube, which is the first-round screened phage sub-library, storing it at -80°C for long-term storage and at -20°C for short-term (1-2 weeks) storage.

3.1.5 Titer Determination of the First-Round Screened Phage Sub-library

The method is the same as that in step 3.1.2 for initial phage titer determination.

3.2 Second-round Screening

3.2.1 Second-round Screening Method

The screening method is the same as that in Step 3.1.1 for the first-round screening. The phage sub-library obtained through amplification and purification in the first-round screening (first-round screening phage sub-library) serves as the input phage library for the second-round screening, resulting in the phage eluate from the second-round screening.

3.2.2 Titer Detection of the Second-round Phage Eluate

The detection method is the same as that in Step 3.1.2 for the initial titer detection of phages.

3.2.3 Amplification and Purification of the Second-round Phage Eluate

The method is the same as those in Steps 3.1.3 and 3.1.4 for the amplification and purification of the first-round phage eluate, resulting in the second-round screening phage sub-library.

3.2.4 Titer Detection of the Second-round Screening Phage Sub-library

The method is the same as that Step 3.1.5 for the titer detection of the first-round screening phage sub-library.

3.3 Third-round Screening

3.3.1 Third-round Screening Method

The screening method is the same as that in Step 3.1.1 for the first-round screening. The phage sub-library obtained through amplification and purification in the second-round screening (second-round screening phage sub-library) serves as the input phage library for the third-round screening, resulting in the phage eluate from the third-round screening.

3.3.2 Titer Detection of the Third-round Phage Eluate

The detection method is the same as that in Step 3.1.2 for the initial titer detection of phages.

3.3.3 Amplification of the Third-round Eluate

The method is the same as that in Step 3.1.3 for the amplification of the first-round phage eluate, resulting in the amplified phage eluate from the third-round.

3.4 Results of the Three Rounds of Screening

The results of the three rounds of affinity screening are shown in Table 2:

Table 2 Results of the Three Rounds of Affinity Screening

| Number of screening rounds | input titer | screening titer | control titer | enrichment level[*1] | fold difference[*2] |
|---|---|---|---|---|---|
| The first round | $1\times10^{12}$ pfu | $2\times10^7$ pfu | $2\times10^6$ pfu | $2\times10^{-5}$ | 10 |
| The second round | $6.6\times10^{12}$ pfu | $3.6\times10^{10}$ pfu | $2\times10^6$ pfu | $5.5\times10^{-3}$ | 18000 |
| The third round | $4.0\times10^{12}$ pfu | $5.2\times10^{10}$ pfu | $6.0\times10^6$ pfu | $1.3\times10^{-2}$ | 8600 |

Here, *1 the enrichment level is calculated by dividing the screening titer by the input titer. A higher value indicates a greater enrichment of the antibody in the input library; *2 the fold difference is calculated by dividing the screening titer by the control titer. A higher value indicates a higher content of positive antibodies in the screening library.
The results showed that after three rounds of screening, the enrichment level reached $1.3\times10^{-2}$ fold, allowing for ELISA monoclonal validation to proceed.

4. Selection of CLL1 VHH Positive Monoclonal Clones

4.1 ELISA Detection of CLL1 VHH Positive Monoclonal Clones

(1) spreading an appropriately diluted bacterial solution from the third-round amplified phage eluate evenly onto a solid medium plate comprising 100 μg/mL of Ampicillin (Amp), and incubating overnight at 37°C;
(2) randomly picking 192 monoclonal colonies and transferring them into a 96-well cell culture plate (P1-P2), adding 200 μL of 2×YT medium (comprising 100 μg/mL of Amp) to each well, and incubating overnight at 37°C;
(3) transferring 5 μL of the overnight-cultured bacterial solution to a new 96-well cell culture plate comprising 200 μL of 2×YT liquid medium (comprising 100 μg/mL of Amp) in each well, and culturing for 5 h at 37°C;
(4) adding helper phage M13K07 at a ratio of bacteria:phage = 1:20 according to the following formula:

$$V\ (mL) = \frac{OD600 \times 1.6 \times 10^{11}}{T_{helper-phage}}$$

wherein V is the volume (in mL) of helper phage added, and $T_{helper-phage}$ is the titer of the helper phage;
(5) incubating at 37°C for 30 min, then adding Kanamycin (Kan) to a final concentration of 50 μg/mL, and culturing overnight at 30°C;

(6) centrifuging the overnight-cultured bacterial solution at 4°C and 4000 rpm for 10 min to obtain the supernatant, which is stored at 4°C for further use;

(7) diluting the screening antigen with PBS to a concentration of 1 $\mu$g/mL, coating the enzyme-linked immunosorbent assay (ELISA) plate with 100 $\mu$L per well, and incubating overnight at 4°C; coating the control wells with 3% Bovine Serum Albumin (BSA);

(8) discarding the antigen solution, adding 200 $\mu$L of PBS buffer to each well, and washing the ELISA plate three times at room temperature for 10 min each time;

(9) adding 200 $\mu$L of blocking solution PBSTB (PBST comprising 2% BSA) to each well to block the ELISA plate, and incubate at room temperature for 1 h.

(10) Discard the blocking solution, add 200 $\mu$L of PBST buffer (1$\times$PBS comprising 0.1% Tween20) to each well, and washing the ELISA plate three times at room temperature for 10 min each time;

(11) adding 100 $\mu$L of blocking solution to each well, then adding 100 $\mu$L of the centrifuged supernatant from step (6) to each well, and incubating at room temperature for 2 h.

(12) discarding the liquid inside the ELISA plate, adding 200 $\mu$L of PBST buffer to each well, and washing three times for 10 min each time;

(13) adding 100 $\mu$L of M13 Bacteriophage HRP Antibody, Mouse Mab (product of Sino Biological, Inc., catalog number 11973-MM05T-H), diluted 1:30000 in blocking solution, to each well, and incubating at room temperature for 1 h;

(14) discarding the liquid inside the ELISA plate, adding 200 $\mu$L of PBST buffer to each well, and washing six times for 5 min each time; and

(15) adding 100 $\mu$L of TMB single-component chromogenic solution to each well, developing the color in the dark for 1-3 min, then adding 100 $\mu$L of 1 M HCl to each well to stop the reaction, and reading the $OD_{450}$ value using a microplate reader.

An $OD_{450}$ value greater than 0.5 is considered a positive result, and the obtained positive clones are phages comprising single-domain antibodies that can bind to the CLL1 protein.

4.2 Secondary ELISA Verification of CLL1 VHH Positive Monoclonal Clones

In order to exclude false-positive results, the clones initially identified as positive were subjected to a secondary ELISA verification, following the same method as described in Step 4.1.

4.3 Sequencing of CLL1 VHH Positive Monoclonal Clones

Five microliters of bacterial solution from 181 positive clones identified in the secondary ELISA verification plate were inoculated into 1 mL of 2$\times$YT medium (comprising 100 $\mu$g/mL of Ampicillin). The cultures were grown at 37°C with shaking at 250 rpm until an $OD_{600}$ of 0.8-1.0 was reached. Subsequently, 0.5 mL of the bacterial solution was taken for sequencing, yielding different antibody sequences.

5. Preparation of Anti-CLL1 Single-Domain Antibodies

5.1 Structure and Sequence of Anti-CLL1 Single-Domain Antibodies

The antibody gene sequences were obtained by sequencing the positive monocl onal clones selected in Step 4.3. Using genetic engineering methods, an anti-CLL1 single-domain antibody (CLL1-VHH) was prepared, with one of the antibodies name d CLL1-VHH-1.

The single-domain antibody consists solely of the variable region (VHH) of the heavy-chain antibody, sequentially composed of framework region FR1, complement arity-determining region CDR1, framework region FR2, complementarity-determining region CDR2, framework region FR3, complementarity-determining region CDR3, a nd framework region FR4. The specific sequence information is as follows.

The amino acid sequence of the single-domain antibody CLL1-VHH-1 is SEQ ID No.1. Among them, positions 1-25 of SEQ ID No.1 constitute framework region FR1, positions 26-35 constitute complementarity-determining region CDR1, position s 36-49 constitute framework region FR2, positions 50-59 constitute complementarity -determining region CDR2, positions 60-98 constitute framework region FR3, positio ns 99-118 constitute complementarity-determining region CDR3, and positions 119-1 29 constitute framework region FR4. The nucleotide sequence of the nucleic acid m olecule encoding the single-domain antibody CLL1-VHH-1 (CLL1-VHH-1 gene) is s hown as SEQ ID No.2.

Among them, positions 1-75 of SEQ ID No.2 constitute the coding sequence f or framework region FR1, positions 76-105 constitute the coding sequence for comp lementarity-determining region CDR1, positions 106-147 constitute the coding sequen ce for framework region FR2, positions 148-177 constitute the coding sequence for complementarity-determining region CDR2, positions 178-294 constitute the coding s equence for framework region FR3, positions 295-354 constitute the coding sequenc e for complementarity-determining region CDR3, and positions 355-387 constitute th e coding sequence for framework region FR4.

The amino acid sequence of the single-domain antibody CLL1-VHH-1 is: 5'-D VQLQESGGGLVQPGGSLRL SCAASGFTFGDYDMTWVRQAPGKGPEWVSAINSGG GSTYYADSVKGRFTISRDNAKNTLYLQMNSLKPEDTAVYYC ATIDRLSTVVAGTYQ PSDEYDYWGQGTQVTVSS-3' (SEQ ID No.1). The three underlined sections repres ent CDR1, CDR2, and CDR3, respectively.

The nucleotide sequence of the CLL1-VHH-1 gene is: 5'-GATGTGCAGCTGCA GGAGTCTGGAGGAGGCT TGGTGCAGCCTGGGGGGTTCTCTGAGACTCTCCTGTG CAGCCTCTGGATTCACCTTCGGAGACTATGACATGACC TGGGTCCGCCAGGCTC CAGGAAAGGGGCCCGAGTGGGTCTCAGCTATTAATAGTGGCGGTGGTAGCACAT ACT ATGCAGACTCCGTGAAGGGCCGATTCACCATCTCCAGAGACAACGCCAAG AACACGCTGTATCTGCAAATGAAC AGCCTGAAACCTGAGGACACGGCCGTGTAT TACTGTGCAACTATCGACCGCCTTAGTACGGTAGTAGCTGGTAC GTACCAGCCGT CCGATGAGTATGACTACTGGGGCCAGGGGACCCAGGTCACCGTCTCCAGC-3' (S EQ ID No.2). The three underlined sections represent the coding sequences for CD R1, CDR2, and CDR3, respectively.

The sequences of the complementarity-determining regions are defined according to the Kabat numbering system.

The aforementioned single-domain antibody specifically binds to the CLL1 prot ein.

5.2 Expression and Purification of Anti-CLL1 Single-Domain Antibodies

The eukaryotic expression vector pcDNA3.1 was used to express the anti-CLL1 single-domain antibody CLL1-VHH-1.

5.2.1 Construction of the Recombinant Vector

In order to facilitate purification, the human IgG1 Fc gene (hFc gene, with nuc leotide sequence SEQ ID No.3 and its encoded amino acid sequence SEQ ID No.4) was added to the 3' end of the coding gene for the single-domain antibody CLL1-VHH-1 (SEQ ID No.2). The resulting fusion gene was named CLL1-VHH-1-hFc. T hen, a *Bam*HI restriction site was added before the start codon at the 5' end of the fusion gene, and an *Eco*RI restriction site was added after the stop codon at the 3' end. The gene was then cloned between the *Bam*HI and *Eco*RI recognition sites o f the eukaryotic expression vector pcDNA3.1, yielding the recombinant eukaryotic e xpression vector pcDNA3.1-CLL1-VHH-1-hFc, which expresses the single-domain an tibody CLL1-VHH-1 fused with hFc (SEQ ID No.4) at the C-terminus.

5.2.2 Expression of Anti-CLL1 Single-Domain Antibodies

The eukaryotic expression vector pcDNA3.1-CLL1-VHH-1-hFc obtained in Step 5.2.1 was transfected into HEK293 cells using conventional electroporation for expre ssion.

5.2.3 Purification of Anti-CLL1 Single-Domain Antibodies

Protein A has the ability to bind to the Fc fragment of IgG; therefore, Protein A columns were used to purify the anti-CLL1 single-domain antibody expressed in HEK293 cells.

The final purified anti-CLL1 single-domain antibody fused with hFc (CLL1-VHH-1-hFc) was obtained.

Example 2: Performance Characterization of Anti-CLL1 Single-Domain Antibodies

1. Determination of Antigen-Antibody Affinity Constant

Test Antibody: the anti-CLL1 single-domain antibody fused with hFc (CLL1-VHH-1-hFc) prepared in Step 5.2.3.

Steps for Determining Affinity Constant:

(1) The test antibody CLL1-VHH-1-hFc was labeled with Pierce™ NHS-Fluorescein Antibody Labeling Kit to obtain the FITC-labeled antibody FITC-CLL1-VHH-1-hFc for flow cytometry detection.

(2) CLL1-positive cells K562-CLL1 and U937 cells were resuspended to a density of $2\times10^6$ cells/mL. A 100 $\mu$L aliquot of the cell suspension was placed in each well of a 96-well V-bottom plate, centrifuged to discard the supernatant, and the cells were collected (individual K562 cells served as CLL1-negative cell controls).

(3) Forty microliters of the single-domain antibody FITC-CLL1-VHH-1-hFc at different gradient dilutions (specific groupings are shown in Table 3) were mixed with the above cells and incubated at room temperature in the dark for 10 min.

Table 3: Groupings for the Affinity Test of the CLL1-VHH-1-hFc Antibody

| | The concentration of antibody (μg/mL) | | | | | | | |
|---|---|---|---|---|---|---|---|---|
| K562-CLL1 | 5.7 | 2.85 | 1.425 | 0.7125 | 0.3563 | 0.1781 | 0.0891 | 0.0445 |
| Antibody dilution ratio | 1:100 | 1:200 | 1:400 | 1:800 | 1:1600 | 1:3200 | 1:6400 | 1:12800 |

(4) Centrifuge and wash twice with 200 μL of FACS buffer, then resuspend in 200 μL of FACS buffer and immediately perform flow cytometry to measure the mean fluorescence intensity (MFI);

(5) Calculate the equilibrium dissociation constant (KD) for the binding of the CLL1 VHH antibody to target cells using the provided formula to determine the strength of the affinity. A smaller KD value indicates stronger affinity.

$$1/(MFI\text{-}Con)=1/Fmax+(KD/Fmax)(1/[CLL1\text{-}scfv\ IgG\ Fc]).$$

Wherein, MFI-Con represents the relative mean fluorescence intensity, which is the value obtained by subtracting the background mean fluorescence intensity from the mean fluorescence intensity of the experimental group; CLL1-scfv IgG Fc represents the amount of antibody used (unit: ng).

Plot a standard curve with the reciprocal of the antibody amount as the x-axis and the reciprocal of the relative mean fluorescence intensity as the y-axis to obtain a linear regression equation. According to the linear regression equation, the intersection of the standard curve with the y-axis is 1/Fmax, and KD is the slope of the line multiplied by Fmax.

The calculated equilibrium dissociation constants are shown in Table 4:

Table 4. Equilibrium dissociation constants (KD) of CLL1-VHH-1-hFc with K562-CLL1 cells

| single-domain antibody | Affinity constant KD (nM) |
|---|---|
| CLL1-VHH-1 | 48.3 |

The results show that the affinity constant between the single-domain antibody (CLL1-VHH-1) obtained by immunizing alpacas and conducting screening in accordance with this disclosure and K562-CLL1 cells is 48.3 nM, indicating a high capacity for binding to the CLL1 protein. This disclosure has successfully screened and obtained the high-affinity anti-CLL1 single-domain antibody, CLL1-VHH-1.

2. Specificity Detection of Single-Domain Antibody CLL1-VHH-1

The expression of CLL1 on different tumor cells was detected using FITC-labeled CLL1-VHH-1 antibody (FITC-CLL1-VHH-1-hFc). The steps are as follows:

(1) CLL1-negative cell lines (control group): K562 cells, U266 cells, and Jurkat cells; CLL1-positive cell lines (experimental group): U937 cells, THP-1 cells, and the CLL1-overexpressing cell line K562-CLL1 were all revived at 37°C and then added to cell culture flasks, wherein, they were cultured at 37°C with 5% $CO_2$ and passaged twice before being used for the detection of the anti-CLL1 single-domain antibody (FITC-CLL1-VHH-1-hFc);

(2) adjusting the cell concentration of each cell line to $2\times10^6$ cells/mL, add 200 μL per well to a 96-well plate, and centrifuge at 1500 rpm for 5 min;

(3) washing with FACS buffer and centrifuge;

(4) adding 20 μL of diluted FITC-CLL1-VHH-1-hFc antibody (1:400), mixing well, and incubating at room temperature for 10 min; and

(5) adding FACS buffer, centrifuging to wash away the antibody, then adding buffer for resuspension, and detecting using a flow cytometer.

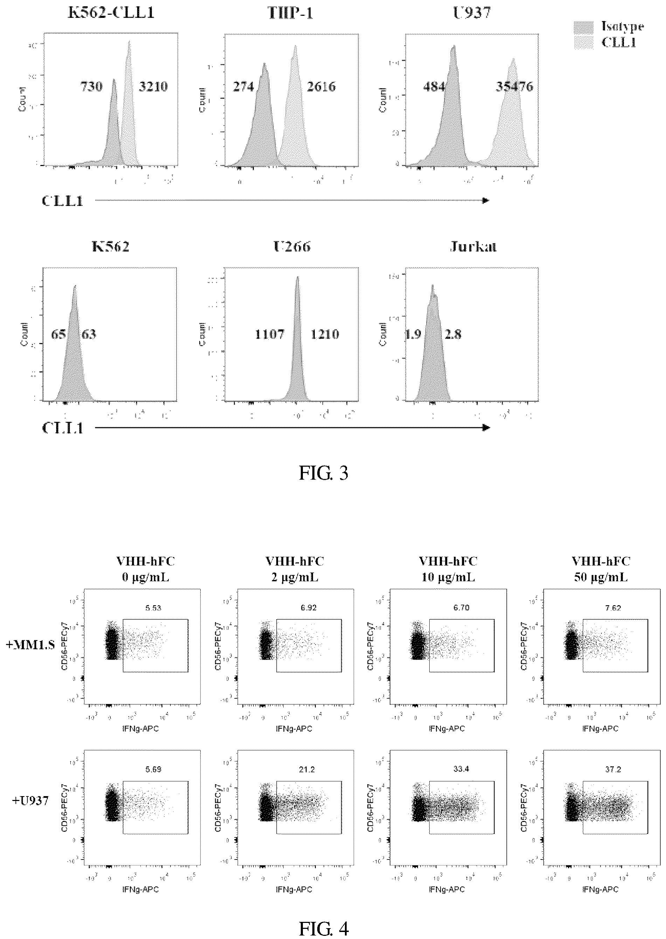

The flow cytometry results showed that the FITC-CLL1-VHH-1-hFc antibody could specifically bind to the CLL1 antigen molecules expressed on tumor cells (FIG. 3).

Example 3: Detection of Antitumor Activity Mediated by Anti-CLL1 Single-Domain Antibody

1. Determination of the Effector Function of NK Cells (IFNγ)

The effector cells were primary PBMCs from healthy donors, calculated based on the percentage of NK cells. The target cells were the CLL1+ cell line U937 and the CLL1- cell line MM1.S. The effector function of NK cells mediated by the anti-CLL1 single-domain antibody was evaluated by measuring the secretion of IFNγ. The determination steps are as follows:

(1) mixing effector cells (NK cells: $1\times10^5/100\ \mu L$) and target cells at a ratio of 1:1, then adding 0, 2, 10, and 50 μg/mL of CLL1-VHH-1 (VHH-hFc) antibody respectively, incubating in a 96-well flat-bottom plate for 4 h;

(2) after the co-incubation, centrifuging the 96-well plate at 1500 rpm for 5 min, removing the supernatant, add 20 μL of diluted anti-human CD3-PE antibody (1:100, Biolegend) and 20 μL of diluted anti-human CD56 PE-Cy7 antibody (1:200, Biolegend), mixing well, and incubating at room temperature for 10 min;

(3) adding 200 μL of FACS buffer for washing, centrifuging, adding 50 μL/well of Cytofix (a membrane-permeabilizing solution, product number 554722 from BD Biosciences) for membrane permeabilization at room temperature for 15 min;

(4) adding 200 μL of Perm buffer for washing twice, centrifuging, then adding 20 μL of diluted anti-human IFNγ-APC antibody (1:50, Biolegend), and incubating at room temperature for 20 min; and

(5) after washing with Perm buffer, detecting the expression of intracellular IFNγ in NK cells by flow cytometry.

The results, as shown in FIG. 4, indicate that the antibody CLL1-VHH-1 (VHH-hFc) of this disclosure can effectively promote the secretion of IFNγ by NK cells and activate the effector function of NK cells. The higher the concentration of the CLL1-VHH-1 antibody, the higher the concentration of IFNγ secreted by NK cells. This shows that the antibody CLL1-VHH-1 of this disclosure can significantly enhance the efficacy of NK cells against CLL1$^+$ target cells, thereby improving antitumor activity.

2. Determination of ADCC Activity Induced by CLL1-VHH-1 Antibody

The effector cells were primary PBMCs from healthy donors, calculated based on the percentage of NK cells. The target cells were the CLL1$^+$ cell line U937 stably expressing Luciferase (U937-Luc cell line) and the CLL1$^-$ cell line MM1.S stably expressing Luciferase (MM1.S-Luc cell line). The determination steps are as follows:

(1) mixing effector cells (NK cells) and target cells ($2\times10^4/100\ \mu L$) at a ratio of 10:1, then adding 6.25, 12.5, 25, and 50 μg/mL of CLL1-VHH-1 (VHH-hFc) antibody respectively, incubating in a 96-well flat-bottom plate for 4 h;

(2) immediately after adding sodium fluorescein (10 μg/mL), measuring the fluorescence value using a TECAN spark microplate reader, calculating the specific cytotoxic activity (Cytotoxicity) of NK cells by taking the average value of three replicate wells.

The analysis formula is:

$$\text{Specific lysis\%} = 100 - 100\times(E_{exp} - E_{min})/(T_{max} - T_{min})$$

$T_{min}$: RLU (Relative luciferase activity) value under the condition of maximum lysis rate;
$E_{exp}$: RLU value when effector cells and target cells are co-cultured;
$T_{max}$: RLU value of spontaneous death of target cells in the absence of effector cells;
$E_{min}$: RLU value of spontaneous death of effector cells in the absence of cells.

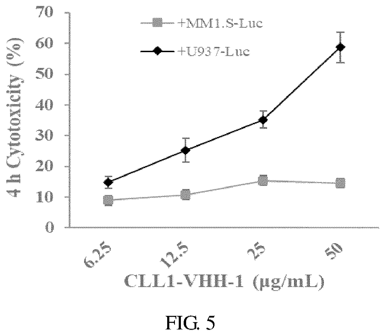

The results, as shown in FIG. 5, indicate that the antibody CLL1-VHH-1 of this disclosure can mediate good ADCC activity. The higher the concentration of the CLL1-VHH-1 antibody, the stronger the specific cytotoxic activity of NK cells. When the concentration of the CLL1-VHH-1 antibody is 50 μg/mL, the specific cytotoxic activity of NK cells can reach 60%. This shows that the antibody CLL1-VHH-1 of this disclosure can significantly induce the cytotoxic effect (ADCC) of NK cells and has a strong ability to specifically kill CLL1-positive cells.

The above has provided a detailed description of this disclosure. For those skilled in the art, without departing from the spirit and scope of this disclosure and without the need for unnecessary experiments, this disclosure can be implemented within a wide range under equivalent parameters, concentrations, and conditions. Although specific examples of this disclosure have been given, it should be understood that further improvements can be made to this disclosure. In short, according to the principles of this disclosure, this application intends to comprise any modifications, uses, or improvements to this disclosure, comprising changes made using conventional techniques known in the field that deviate from the scope already disclosed in this application. Based on the scope of the following accompanying claims, some basic features can be applied.

## Industrial Applications

The anti-CLL1 single-domain antibody (CLL1-VHH-1) of this disclosure exhibits high affinity and excellent specificity, enabling it to effectively and specifically target CLL1-positive cells (cells expressing the CLL1 protein). The antibody CLL1-VHH-1 of this disclosure can more efficiently promote the secretion of IFNγ by NK cells, activate the effector functions of NK cells, enhance the efficacy of NK cells against CLL1+ target cells, thereby improving anti-tumor activity. Additionally, it can significantly induce the cytotoxic effects of NK cells (ADCC), demonstrating a strong capacity for specifically killing CLL1-positive cells. The single-domain antibody of this disclosure can be formulated into specific antibody drugs for the clinical prevention and treatment of diseases associated with the CLL1 target (such as acute myeloid leukemia, myelodysplastic syndromes, or chronic myeloid leukemia). It can also be utilized in the preparation of CAR cells targeting CLL1 or in the development of detection kits for the CLL1 protein. The single-domain antibody drug of this disclosure features stable structure, small molecular size, ease of recombinant expression, and low production costs. It can be used alone or serve as a drug delivery system to carry relevant drugs, holding immense potential and significant importance in the fields of pharmaceutical uses and clinical diagnostics.

## Claims

1. A single-domain antibody, wherein the single-domain antibody comprises a heavy-chain variable region, and the heavy-chain variable region comprises a complementarity-determining region CDR1 with the amino acid sequence being positions 26-35 of SEQ ID No.1, a complementarity-determining region CDR2 with the amino acid sequence being positions 50-59 of SEQ ID No.1, and a complementarity-determining region CDR3 with the amino acid sequence being positions 99-118 of SEQ ID No.1.

2. The single-domain antibody according to claim 1, wherein the amino acid sequence of the heavy-chain variable region is SEQ ID No.1 or an amino acid sequence that has at least 80% identity to SEQ ID No.1 and has the same function.

3. A biological material related to the single-domain antibody according to claim 1 or 2, wherein the biological material is any one of the following C1) to C4):

   c1) a nucleic acid molecule encoding the heavy-chain variable region of the single-domain antibody according to claim 1 or 2;
   c2) an expression cassette comprising the nucleic acid molecule described in C1);
   c3) a recombinant vector comprising the nucleic acid molecule described in C1) or a recombinant vector comprising the expression cassette described in C2);
   c4) a recombinant host cell comprising the nucleic acid molecule described in C1), or a recombinant host cell comprising the expression cassette described in C2), or a recombinant host cell comprising the recombinant vector described in C3).

4. The biological material according to claim 3, wherein the nucleic acid molecule is any one of the following:

   d1) a DNA molecule whose nucleotide sequence or coding sequence is SEQ ID No.2;
   d2) a DNA molecule that has 75% or more identity to the nucleotide sequence defined in D1) and has the same function.

5. Any one of the following uses of the single-domain antibody according to claim 1 or 2 or the biological material according to claim 3 or 4:

   E1) a use in preparation of a drug for preventing or treating tumors, or use in preventing or treating tumors;
   E2) a use in preparation of a drug for preventing or treating diseases related to the CLL1 target, or use in preventing or treating diseases related to the CLL1 target;
   E3) a use in preparation of a product for screening, diagnosing, or aiding in the diagnosis of diseases related to the CLL1 target, or use in screening, diagnosing, or aiding in the diagnosis of diseases related to the CLL1 target;
   E4) a use in detecting a CLL1 protein or in preparation of a product for detecting the CLL1 protein;
   E5) a use in preparation of a product for binding to the CLL1 protein;
   E6) a use in mediating a specific recognition of tumors expressing a CLL1 antigen by drugs or in preparation of a product for mediating the specific recognition of tumors expressing the CLL1 antigen by drugs;
   E7) a use in in vivo imaging of the CLL1 protein or in preparation of a product for in vivo imaging of the CLL1 protein;
E8) a use in preparation of a product targeting the CLL1.

6. The use according to claim 5, wherein the diseases related to the CLL1 target are CLL1-positive cancers.

7. The use according to claim 6, wherein the CLL1-positive cancers are acute myeloid leukemia, myelodysplastic syndrome, or chronic myeloid leukemia.

8. A pharmaceutical composition, wherein the pharmaceutical composition comprises the single-domain antibody according to claim 1 or 2, or chimeric antigen receptor-modified cells, where the chimeric antigen receptor comprises the single-domain antibody according to claim 1 or 2.

9. A reagent or kit, wherein the reagent or kit comprises the single-domain antibody according to claim 1 or 2, and the reagent or kit has any one of the following uses:

g1) detecting a CLL1 protein;
g2) screening, diagnosing, or aiding in diagnosis of diseases related to the CLL1 target;
g3) in vivo imaging of the CLL1 protein.

10. A preparation method for the single-domain antibody according to claim 1 or 2, wherein the method comprises: constructing a recombinant expression vector comprising the nucleic acid molecule according to claim 3 or 4; introducing the recombinant expression vector into host cells to obtain recombinant cells expressing the single-domain antibody; culturing the recombinant cells and obtaining the single-domain antibody through separation and purification.

11. A method for diagnosing or aiding in diagnosis of diseases related to a CLL1 target, wherein the method comprises: isolating a sample from a subject, and then detecting content of a CLL1 protein in the sample using the single-domain antibody according to claim 1 or 2, and performing the diagnosis or aiding in the diagnosis of diseases related to the CLL1 target based on the content of the CLL1 protein.

12. A method for screening diseases related to CLL1 target, wherein the method comprises: isolating a sample from a subject, and then detecting content of a CLL1 protein in a sample using the single-domain antibody according to claim 1 or 2, and performing the screening of diseases related to the CLL1 target based on the content of the CLL1 protein.

13. A method for preventing or treating diseases related to a CLL1 target, wherein the method comprises administering the single-domain antibody according to claim 1 or 2 or the pharmaceutical composition according to claim 8 to a subject suffering from diseases related to the CLL1 target.

14. The method according to any one of claims 11-13, wherein the diseases related to the CLL1 target are tumors related to the CLL1 target.

15. The method according to claim 14, wherein the tumors related to the CLL1 target are CLL1-positive cancers.

16. The method according to claim 15, wherein the CLL1-positive cancers are acute myeloid leukemia, myelodysplastic syndrome, or chronic myeloid leukemia.

17. A method for in vitro detection of a CLL1 protein, wherein the method comprises detecting a CLL1 protein using the single-domain antibody according to claim 1 or 2 or the reagent or kit according to claim 9.

18. An antibody-drug conjugate, comprising an antibody moiety and a conjugated moiety, wherein the antibody moiety comprises the single-domain antibody according to claim 1 or 2.

19. The antibody-drug conjugate according to claim 18, wherein the conjugated moiety comprises a detectable label, a chemical drug, or a cytotoxin.

FIG. 1

FIG. 2

FIG. 3

FIG. 4

FIG. 5

## INTERNATIONAL SEARCH REPORT

| International application No. |
| --- |
| **PCT/CN2023/084931** |

| A. | CLASSIFICATION OF SUBJECT MATTER |
| --- | --- |

C07K16/28(2006.01)i; C12N15/13(2006.01)i; C12N15/85(2006.01)i; C12N5/10(2006.01)i; A61K47/68(2017.01)i; A61K49/00(2006.01)i; A61K51/10(2006.01)i; G01N33/574(2006.01)i

According to International Patent Classification (IPC) or to both national classification and IPC

| B. | FIELDS SEARCHED |
| --- | --- |

Minimum documentation searched (classification system followed by classification symbols)

IPC: C07K; C12N; A61K; G01N

Documentation searched other than minimum documentation to the extent that such documents are included in the fields searched

Electronic data base consulted during the international search (name of data base and, where practicable, search terms used)

Databases: CNTXT, ENTXTC, ENTXT, DWPI, WOTXT, WOTXTC, VEN, WPABS, WPABSC, CJFD, CNKI, 万方, WANFANG, 百度学术, Baidu Scholar, PubMed, ISI_Web of Science, 中国专利生物序列检索, China Patent Biological Sequence Search, STN, GenBank, EMBL-EBI; search terms: C-凝集素样分子, C型激素结构域家族12成员A, 单域抗体, 重链抗体, 抗体, 纳米抗体, 表达盒, 宿主细胞, 肿瘤, 抗体偶联物, 急性髓细胞白血病, 慢性髓细胞白血病, 骨髓增生异常综合征, CLEC12A, CLL1, C-type lectin-like molecule, C-type lectin domain family 12 member A, nanobody, sdAb, VHH, single domain antibody, heavy chain antibody, HcAb, Nb, AINSGGGSTY, GFTFGDYDMT, IDRLSTVVAGTYQPSDEYDY, expression cassette, host cell, vector, cancer, tumor, tumour, ADC, AML, acute myeloid leukemia, CML, chronic leukemia, MDS, myelodysplastic syndromes, 对SEQ ID NOs: 1-4进行检索, search for SEQ ID NOs: 1-4.

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 114127115 A (INHIBRX LP) 01 March 2022 (2022-03-01) <br> see claims 1-42, and description, paragraphs 4-6 | 1-19 |
| A | CN 113039205 A (CARSGEN THERAPEUTICS CO., LTD.) 25 June 2021 (2021-06-25) <br> see claims 1-29, and description, embodiments 1-5 | 1-19 |
| A | WO 2023006117 A1 (NANJING LEGEND BIOTECH CO., LTD.) 02 February 2023 (2023-02-02) <br> see claims 1-79, and description, paragraphs 8-23 | 1-19 |
| A | US 2022396626 A1 (GUANGZHOU BIO GENE TECH CO., LTD.) 15 December 2022 (2022-12-15) <br> see claims 1-15, and description, paragraphs 5-51 | 1-19 |

☑ Further documents are listed in the continuation of Box C.     ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **12 June 2023** | **04 July 2023** |

| Name and mailing address of the ISA/CN | Authorized officer |
| --- | --- |
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/084931**

| | C. DOCUMENTS CONSIDERED TO BE RELEVANT | |
|---|---|---|
| Category* | Citation of document, with indication, where appropriate, of the relevant passages | Relevant to claim No. |
| A | US 2022354890 A1 (LI GUANGCHAO) 10 November 2022 (2022-11-10)<br>see claims 1-15, and description, paragraphs 4-49 | 1-19 |

**INTERNATIONAL SEARCH REPORT**

International application No.

**PCT/CN2023/084931**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2023/084931**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **5-7, 13-16**
   because they relate to subject matter not required to be searched by this Authority, namely:

   PCT Rule 39.1(iv) – methods for treatment of the human or animal body by surgery or therapy.

   The search for claims 5-7 is made on the basis of the following reasonably expected subject matter: any one of the following uses of the single-domain antibody of claim 1 or 2 or the biological material of claim 3 or 4: E1) the use in the preparation of a drug for preventing or treating tumors; E2) the use in the preparation of a drug for preventing or treating CLL1 target-related diseases; E3) the use in the preparation of a product for screening, diagnosing or assisting in diagnosing CLL1 target-related diseases, or the use in in-vitro screening, diagnosis or auxiliary diagnosis of CLL1 target-related diseases; E4) the use in detecting CLL1 protein in vitro or preparing a product for detecting CLL1 protein; E5) the use in the preparation of a product for binding CLL1 protein; E6) the use in the preparation of a product for mediating a drug to specifically recognize a tumor expressing CLL1 antigen; E7) the use in the preparation of a product for in vivo imaging of CLL1 protein; and E8) the use in the preparation of a product targeting CLL1.

   The search for claims 13-16 is made on the basis of the following reasonably expected subject matter: the use of the single-domain antibody of claim 1 or 2 or the pharmaceutical composition of claim 8 in the preparation of a drug for preventing or treating CLL1 target-related diseases.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/084931**

| Patent document cited in search report | | | Publication date (day/month/year) | Patent family member(s) | | | Publication date (day/month/year) |
|---|---|---|---|---|---|---|---|
| CN | 114127115 | A | 01 March 2022 | CA | 3138972 | A1 | 12 November 2020 |
| | | | | JP | 2022531306 | A | 06 July 2022 |
| | | | | WO | 2020227073 | A1 | 12 November 2020 |
| | | | | US | 2020347139 | A1 | 05 November 2020 |
| | | | | AU | 2020267349 | A1 | 11 November 2021 |
| | | | | KR | 20220004751 | A | 11 January 2022 |
| | | | | EP | 3966243 | A1 | 16 March 2022 |
| | | | | IL | 287781 | A | 01 January 2022 |
| | | | | SG | 11202111731 | WA | 29 November 2021 |
| CN | 113039205 | A | 25 June 2021 | SG | 11202104240 | TA | 28 May 2021 |
| | | | | WO | 2020083406 | A1 | 30 April 2020 |
| | | | | CA | 3117759 | A1 | 30 April 2020 |
| | | | | EP | 3875484 | A1 | 08 September 2021 |
| | | | | EP | 3875484 | A4 | 20 July 2022 |
| | | | | KR | 20210089179 | A | 15 July 2021 |
| | | | | US | 2021324087 | A1 | 21 October 2021 |
| | | | | JP | 2022505921 | A | 14 January 2022 |
| WO | 2023006117 | A1 | 02 February 2023 | None | | | |
| US | 2022396626 | A1 | 15 December 2022 | GB | 202207720 | D0 | 06 July 2022 |
| | | | | GB | 2610467 | A | 08 March 2023 |
| | | | | AU | 2020480236 | A1 | 28 July 2022 |
| | | | | JP | 2023509821 | A | 10 March 2023 |
| | | | | KR | 20220084013 | A | 21 June 2022 |
| | | | | EP | 4039708 | A1 | 10 August 2022 |
| | | | | WO | 2022120943 | A1 | 16 June 2022 |
| US | 2022354890 | A1 | 10 November 2022 | WO | 2022120942 | A1 | 16 June 2022 |
| | | | | GB | 202207722 | D0 | 13 July 2022 |
| | | | | GB | 2606869 | A | 23 November 2022 |
| | | | | EP | 4039712 | A1 | 10 August 2022 |
| | | | | JP | 2023510465 | A | 14 March 2023 |
| | | | | KR | 20220084040 | A | 21 June 2022 |
| | | | | AU | 2020480789 | A1 | 04 August 2022 |

Form PCT/ISA/210 (patent family annex) (July 2022)